# FASCICULE DE BREVET EUROPEEN

(11) **EP 0 641 557 B1**
(45) Date de publication et mention de la délivrance du brevet: **21.08.1996**
(21) Numéro de dépôt: 94401880.3
(22) Date de dépôt: 22.08.1994
(51) Int. Cl.: A61K 7/00, A61K 9/127

(54) **Composition cosmétique ou dermatologique constituée d'une émulsion huile dans eau à base de globules huileux pourvus d'un enrobage cristal liquide lamellaire**
Kosmetische und dermatologische Zusammensetzung bestehend aus einem öligen Kügelchen und mit lamellaren Flüssigkristallen beschichteter Öl in Wasser Emulsion
Dermatologic or cosmetic composition made up by an oil in water emulsion based on oily globules coated with a lamellar liquid crystal coating

(30) Priorité: 07.09.1993 FR 9310588
(43) Date de publication de la demande: 08.03.1995
(73) Titulaire: L'OREAL, F-75008 Paris (FR)
(72) Inventeur: Ribier, Alain, F-75001 Paris (FR); Simonnet, Jean-Thierry, F-75011 Paris (FR); Griat, Jacqueline, F-94480 Ablon (FR)
(74) Mandataire: Lhoste, Catherine

(56) Documents cités:
- EP-A- 0 466 236
- EP-A- 0 512 270
- GB-A- 2 139 112
- DATABASE WPI Section Ch, Week 8903, Derwent Publications Ltd., London, GB; Class A12, AN 89-018044 & JP-A-63 287 718 (SHISEIDO KK) 24 Novembre 1988
- J. PHARM. SCIENCES, vol.78, no.8, Août 1989, WASHINGTON pages 683 - 687, XP142848 HUSSEIN G.I. 'RELEASE STUDIES FROM LYOTROPIC LIQUID CRYSTAL SYSTEMS'
- DATABASE WPI Section Ch, Week 9038, Derwent Publications Ltd., London, GB; Class D08, AN 90-286002 & JP-A-2 200 608 (POLA CHEM KK ET AL) Août 1990
- CHEMICAL ABSTRACTS, vol. 117, no. 4, 27 Juillet 1992, Columbus, Ohio, US; abstract no. 33422b, TSUDA H. ET AL 'TRANSPARENT OR SEMI TRANSPARENT HAIR PREPARATIONS CONTAINING SURFACTANTS, OILS AND/OR FATS AND WATER SOLUBLE ALCOHOLS'

## Description

La présente invention concerne une composition cosmétique ou dermatologique constituée par une émulsion du type huile dans eau. Elle concerne plus particulièrement une composition constituée par une émulsion huile dans eau formée par des globules huileux pourvus d'un enrobage cristal liquide lamellaire dispersés dans une phase aqueuse. L'invention concerne également un procédé de préparation d'une telle composition et son application au traitement de la peau et de la matière kératinique.

De nombreux composés actifs lipophiles ont un rôle important à jouer dans les domaines du soin de la peau. On peut citer comme exemples de tels actifs les vitamines lipophiles A, E ou F, les huiles essentielles, les filtres antisolaires, les alkylesters à longue chaîne d'α-hydroxyacides, les antiinflammatoires, ainsi que les agents biostimulants des synthèses de lipides et/ou de protéines.

Leur utilisation est très répandue mais leur efficacité se voit limitée de par leur nature lipophile. Ils ne sont en effet que très partiellement absorbés par la peau et ne diffusent que très difficilement dans les assises du stratum corneum dans lesquelles ils sont arrêtés par la présence de compartiments aqueux au sein des espaces intercornéocytaires.

Leur introduction dans des émulsions huile dans eau stabilisées par une monocouche de tensioactifs n'améliore pratiquement pas cet état de fait étant donné que ces émulsions se cassent dès leur application sur la peau en libérant à la surface de la peau une phase huileuse contenant les actifs lipophiles qui sont très mal absorbés pour les raisons évoquées ci-dessus.

Des tentatives d'amélioration de cet état de fait ont été proposées dans l'art antérieur.

Dans JSCC 35, 45-57 (Janvier, Février 1984), JUNGINGER et al décrivent des émulsions huile dans eau dont la stabilisation est assurée par un réseau tridimentionnel cristal liquide lamellaire.

SUZUKI et al dans "Secondary droplet emulsion : Contribution of liquid crystal formation to physicochemical properties and skin moisturizing effect of cosmetic emulsion" (12ème Congrès international IFSSC, Paris septembre 1992, Abstracts, Vol I, 117-136) décrivent ces émulsions huile dans eau comme formant des superstructures ("secondary droplets"), des agrégats de gouttelettes huileuses enrobées de lamelles de cristaux liquides. Ces auteurs montrent que l'existence de ces superstructures est dépendante de la présence d'alcool gras.

Ce type d'émulsions a pour qualités principales la stabilité vis à vis des lâchages d'huile ainsi qu'un effet hydratant sur la peau ; il présente également des désavantages. Il est, en effet, nécessaire de mettre en oeuvre de grandes quantités de tensioactif pour constituer le réseau tridimentionnel ce qui augmente les risques d'intolérance de la part de l'utilisateur et ce qui se traduit par un long "savonnage" (persistance d'une coloration blanche) lors de l'application sur la peau de telles compositions. De plus, la dispersion d'huile est grossière, hétérogène et l'huile est davantage sequestrée par le réseau tridimentionnel que réellement dispersée sous forme de microgouttelettes d'huile individualisées. Les gouttelettes d'huile générées par ce type d'émulsion ont une taille moyenne très supérieure aux espaces intercornéocytaires qu'elles ont à traverser et aux pores des cheveux qu'elles ont à investir, ce qui contribue à expliquer la pénétration très partielle, dans la peau et le cheveu, de la phase grasse et des actifs qui y sont dissous.

On connait encore l'article de DAHMS dans Cosmectics and Toiletries, Vol 101 Novembre 1986 qui décrit des émulsions présentant les mêmes caractéristiques et, donc les mêmes inconvénients.

On constate donc que subsistait dans l'art antérieur le besoin d'émulsions permettant une pénétration améliorée des compositions cosmétiques ou dermatologiques dans la peau et dans le cheveu.

La demanderesse a maintenant découvert une nouvelle émulsion permettant d'atteindre cet objectif.

La présente demande a donc pour objet une composition cosmétique ou dermatologique constituée par une émulsion du type huile dans eau formée de globules huileux pourvus chacun d'un enrobage cristal liquide lamellaire et dispersés dans une phase aqueuse, caractérisée en ce que chaque globule huileux contenant au moins un composé actif lipophile cosmétique ou dermatologique est unitairement enrobé d'une couche monolamellaire ou oligolamellaire obtenue à partir d'au moins un agent tensioactif lipophile, d'au moins un agent tensioactif hydrophile et d'au moins un acide gras, les globules huileux enrobés ayant un diamètre moyen inférieur à 500 nanomètres, et en ce que la phase aqueuse contient à l'état dissous un agent basique.

La présente invention a aussi pour objet une émulsion du type huile dans eau formée de globules huileux pourvus chacun d'un enrobage cristal liquide lamellaire et dispersés dans une phase aqueuse, caractérisée en ce que chaque globule huileux est unitairement enrobé d'une couche monolamellaire ou oligolamellaire obtenue à partir d'au moins un agent tensioactif lipophile, d'au moins un agent tensioactif hydrophile et d'au moins un acide gras, les globules huileux enrobés ayant un diamètre moyen inférieur à 500 nanomètres, et en ce que la phase aqueuse contient à l'état dissous un agent basique.

Au sens de la présente invention, on entend par composé actif lipophile le composé actif en soi lorsqu'il est lui-même une huile ou bien, s'il ne l'est pas, le composé actif dissous dans une huile. Les huiles pouvant être utilisées sont les huiles servant classiquement de support dans les compositions cosmétiques comme par exemple les triglycérides d'acides gras à chaînes courtes, les huiles de silicone, etc.

L'invention permet de disposer d'émulsions ayant des gouttelettes de phase grasse de taille extrèmement petite revêtues d'une couche mono ou oligo lamellaire extrèmement fine. On entend par couche oligolamellaire une couche comprenant de 2 à 5 lamelles lipidiques. La taille moyenne des globules huileux revêtus étant inférieure à 500 nanomètres et, de préférence, à 200 nanomètres, leur pénétration dans les espaces intercornéocytaires qui sont de taille comparable est grandement facilitée.

L'actif contenu ou constitué par le globule huileux peut de ce fait être véhiculé et délivré dans la peau ou le cheveu là ou son action sera la plus efficace.

Selon un mode de réalisation préférentiel de l'invention l'agent tensioactif lipophile, l'agent tensioactif hydrophile et l'acide gras comportent chacun au moins une chaine grasse saturée ayant plus de 12 atomes de carbone environ. Plus préférentiellement encore, cette chaine grasse contient de 16 à 22 atomes de carbone.

Selon un autre mode de réalisation préférentiel de l'invention, l'agent tensioactif lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.

Des exemples de tels agents tensioactifs lipophiles sont le distéarate de sucrose, le distéarate de diglycéryle, le tristéarate de tétraglycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE (comportant 2 motifs oxyéthylène), le mono et dibéhénate de glycéryle, le tétrastéarate de pentaérythrytol.

L'agent tensioactif hydrophile présente de préférence un HLB compris entre environ 8 et environ 12.

On peut citer comme exemples de tels tensioactifs hydrophiles les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE et le distéarate polyoxyéthylèné 12 OE, le distéarate de méthylglucose polyoxyéthyléné 20 OE.

Comme cela a été dit plus haut, l'acide gras saturé mis en oeuvre dans le cadre de la présente invention est de préférence un acide gras saturé ayant de 16 à 22 atomes de carbone. On préfère ainsi utiliser l'acide palmitique, l'acide stéarique, l'acide arachidique et l'acide béhénique.

L'enrobage selon l'invention des globules huileux demande de préférence l'utilisation d'une quantité totale d'agent tensioactif hydrophile, d'agent tensioactif lipophile et d'acide gras comprise entre environ 2% et environ 6% en poids par rapport au poids total de la composition. Encore plus préférentiellement, cette quantité est comprise entre 3% et 4%. Les quantités relatives de tensioactif lipophile, hydrophile et d'acide gras varient de préférence dans les fourchettes respectives suivantes : 35-55% / 25-40% / 15-35% en poids par rapport à leur poids total.

La phase grasse, c'est à dire les gouttelettes huileuses enrobées, représente de préférence 5 à 50% en poids par rapport au poids total de la composition. Encore plus préférentiellement ce pourcentage est compris entre 10 et 40. De préférence, le rapport huile/eau est égal ou inférieur à 1.

Le rapport pondéral des globules huileux aux éléments constitutifs de l'enrobage est de préférence de 2 à 13 ; encore plus préférentiellement ce rapport est égal à 7 environ.

L'agent basique contenu dans la phase aqueuse des compositions selon l'invention est destiné à la neutralisation de l'acide gras présent dans la phase huileuse. Il doit donc être présent en une quantité au moins égale à celle nécessaire à la neutralisation de la totalité de l'acide gras.

On peut utiliser comme agent basique, par exemple, la soude, la triéthanolamine, la lysine ou bien encore l'arginine.

Lorsque les compositions selon l'invention sont utilisées pour le traitement cosmétique de la peau ou dans un but dermatologique l'actif contenu dans la phase huileuse est, par exemple, choisi parmi les agents antioxydants, anti radicaux libres, hydratants, mélanorégulateurs, accélérateurs de bronzage, dépigmentants, de coloration de la peau, liporégulateurs, amincissants, anti-acnéiques, antiséborrhéiques, anti-vieillissement, anti-rides, anti-UV, kératolytiques, anti-inflammatoires, rafraichissants, cicatrisants, protecteurs vasculaires, antibactériens, antifungiques, antiperspirants, déodorants, conditionneurs de la peau, immunomodulateurs, nourrissants et les huiles essentielles et les parfums.

Lorsque les compositions selon l'invention sont utilisées pour le traitement cosmétique de la matière kératinique, l'actif contenu dans la phase huileuse est, par exemple, choisi parmi les agents mélanorégulateurs, liporégulateurs, antiséborrhéiques, anti-vieillissement, anti-UV, kératolytiques, antibactériens, antifungiques, antipelliculaires, anti-chute des cheveux, colorants capillaires, décolorants capillaires, réducteurs pour permanente, conditionneurs des cheveux et nourrissants.

On peut citer comme exemples d'actifs lipophiles pour le traitement de la peau et/ou des cheveux, utilisables dans le cadre de la présente invention les composés suivants :

D α tocophérol, DL α tocophérol, acétate de D α tocophérol, acétate DL α tocophérol, palmitate d'ascorbyle, glycérides de vitamine F, vitamines D, vitamine D₂, vitamine D₃, rétinol, esters de rétinol (palmitate de rétinol, propionate de rétinol), β carotène, D panthénol, farnesol, acétate de farnesyle, huiles de jojoba, de cassis riches en acides gras essentiels, acide n octanoyl-5 salicylique, acide salicylique, alkylesters d'αhydroxyacides tels que acide citrique, acide lactique, acide glycolique, acide asiatique, acide madécassique, asiaticoside, extrait total de Centella asiatica, acide β glycyrrhétinique, α bisabolol, céramides comme le 2 oleoylamino-1,3 octadécane, phytanetriol, sphingomyéline de lait, phospholipides d'origine marine riches en acides gras essentiels polyinsaturés, éthoxyquine, extrait de romarin, extrait de mélisse, quercetine, extrait de microalgues séchées (algoxan red de Algatec), huile essentielle de bergamotte, octylmethoxycinnamate (Parsol MCX - Givaudan-Roure), butylméthoxydibenzoylméthane (Parsol 1789 - Givaudan-Roure), octyl triazone (Uvinul T150 - BASF), oxydes de fer jaune, brun, noir, rouge, oxydes de titane, pouvant se présenter sous forme micrométrique ou nanométrique ou sous forme enrobée (perfluoroalkyl), di-tertiobutyl-3,5 hydroxy-4 benzylidène 3 camphre, 2-benzotriazol -2-yl- 4-méthyl-6-[3-[1,3,3,3-tétraméthyl-1 - [(triméthylsilyl)oxy]-disiloxanyl]-2-méthyl-propyl]phénol, huile perfluorée (perfluorodécaline, perfluorooctyl bromure), huile de maïs hyperoxygénée (Epaline 100 commercialisée par la société Carilene).

Les compositions selon la présente invention peuvent en outre contenir dans la phase aqueuse un ou plusieurs composés hydrophiles cosmétiquement ou dermatologiquement actifs, libres ou encapsulés.

On peut utiliser les actifs hydrophiles classiquement utilisés comme agents antioxydants, anti radicaux libres, hydratants, mélanorégulateurs, accélérateurs de bronzage, dépigmentants, de coloration de la peau, liporégulateurs, amincissants, anti-acnéiques, antiseborrheiques, anti-vieillissement, anti-rides, anti-UV (acide benzène-1,4-[di(3-méthylidènecampho-10-sulfonique)], kératolytiques, anti-inflammatoires, rafraichissants, cicatrisants, protecteurs vasculaires, antibactériens, antifongiques, antiperspirants, déodorants, conditionneurs de la peau et du cheveu, immunomodulateurs, nourrissants, antipelliculaires, anti-chute des cheveux, colorants capillaires, décolorants capillaires, réducteurs pour permanente et les huiles essentielles et les parfums.

Lorsqu'il se présente à l'état encapsulé ce composé peut être incorporé dans un vésicule lipidique obtenu à partir de lipides ioniques, non ioniques ou d'un mélange des deux. Il peut encore être incorporé dans des nanoparticules lipidiques telles que les nanosphères, nanoéponges ou nanocapsules.

L'incorporation de vésicules lipidiques dans les compositions de l'invention est particulièrement avantageuse du fait de la complémentarité et de la bonne compatibilité de ces deux types de véhicules que sont, d'une part, les globules huileux contenant des actifs lipophiles, de taille moyenne préférée voisine de 200 nm, délimités par leur enrobage cristal liquide lamellaire et, d'autre part, les vésicules lipidiques à coeur aqueux contenant des actifs hydrophiles, de taille moyenne préférée voisine de 200 nm, délimités par leur paroi lamellaire.

Les inventeurs ont de plus constaté que les vésicules lipidiques à base de lipides ioniques naturels insaturés, qui sont particulièrement sensibles à la présence de tensioactifs en phase aqueuse (ce qui est le cas dans les émulsions classiques) et à celle des péroxydes étaient particulièrement bien conservées dans les compositions de l'invention à base de tensioactifs à chaînes grasses saturées ayant plus de 12 atomes de carbone.

Les compositions de l'invention peuvent encore contenir dans la phase aqueuse divers additifs complémentaires tels que des agents conservateurs, des agents sequestrants, des agents gélifiants...

Les compositions de l'invention peuvent encore contenir dans la phase grasse divers additifs complémentaires tels que huiles, cires ou gommes ayant par exemple des propriétés emollientes ou lubrifiantes.

Les compositions se présentent le plus fréquemment sous forme de lait, crème ou gel, d'autres modes de présentation n'étant pas exclus.

Un autre objet de l'invention est un procédé de préparation des compositions ci-dessus décrites caractérisé en ce que dans une première étape on mélange sous agitation la phase grasse comprenant le tensioactif lipophile, le tensioactif hydrophile, l'acide gras, le composé actif au plan cosmétique ou dermatologique et la phase aqueuse comprenant l'agent neutralisant et dans une deuxième étape on soumet le mélange obtenu à une homogénéisation basée sur le principe de la cavitation.

Dans la première étape le mélange est soumis à agitation classique par exemple dans un homogénéisateur tournant à une vitesse comprise entre 500 et 5000 t/mn environ pendant un temps compris entre 10 et 60 mn environ à une température comprise entre 20 et 95°C environ.

L'homogénéisation basée sur le principe de la cavitation de la deuxième étape est une étape clé du procédé selon l'invention. Cette homogénéisation résulte du phénomène de cavitation créé et entretenu au sein du mélange, alors sous forme liquide, en mouvement à une vitesse linéaire d'au moins 100m/s.

Elle peut être opérée par utilisation d'un homogénéisateur haute pression fonctionnant sous des pressions comprises entre 200 et 1000 bars environ. Le principe d'utilisation de ce type d'homogénéisateur est bien connu de l'homme de l'art. On opère par passage successifs, généralement de 2 à 10 passages, sous la pression retenue, le mélange étant ramené à pression normale entre chaque passage.

L'homogénéisation de la deuxième étape peut également être obtenue sous l'action d'ultrasons ou encore par utilisation d'homogénéisateurs équipés d'une tête de type rotor-stator.

Lorsque la phase aqueuse contient des actifs hydrophiles actifs au plan cosmétique ou dermatologique, si ces derniers sont introduits à l'état libre ils sont introduits dans la première étape. Si par contre ils sont introduits à l'état encapsulés ils doivent être introduits dans une troisième étape ultérieure. Ils le sont alors par simple mélange.

L'invention va être maintenant plus complètement décrite, dans ses objets et ses caractéristiques, à l'aide des exemples qui vont suivre.

On opère dans ces exemples en mettant en oeuvre le mode opératoire suivant :

La phase huileuse A1 et la phase aqueuse B sont chauffées séparément à la température de 80° C.

Sous l'agitation de 4000t/mn fournie par un homogénéisateur Moritz de type Turbo Lab 2100, on verse la phase B sur la phase A et l'on conserve ces conditions d'agitation et de tempéraure pendant 30 minutes.

Le mélange est alors introduit dans un homogénéisateur haute pression Soavi de type OBL réglé à la pression de 500 bars pour 3 passages consécutifs.

On obtient ainsi une émulsion huile dans eau stabilisée, dont les globules huileux ont une taille moyenne inférieure à 200 nm et une polydispersité d'indice inférieure à 0,1 telles que mesurées par un granulomètre à laser de type AMTECH BI 90.

On refroidit ensuite l'émulsion pour la ramener à température ambiante, ce qui prend environ 60 minutes. On ajoute alors à l'émulsion la phase huile A2 et on soumet le tout à l'agitation donnée par le Turbo Lab 2100 à la vitesse de 3000 t/mn pendant 10 mn, après quoi on introduit ce prémélange dans le Soavi - OBL réglé à la pression de 350 bars pour 2 passages supplémentaires.

Après chacun de ces deux passages, on refroidit le produit jusqu'à retour à la température ambiante.

A cette émulsion A1 + B + A2, on ajoute la phase C et l'on agite le tout à l'aide d'un homogénéisateur Rayneri équipé d'une turbine de type défloculeuse à la vitesse de 2500 t/mn pendant 30 mn à la température ambiante.

### EXEMPLE 1 : Crème de jour pour le visage, destinée aux peaux sèches.

### Phase A1 :

| | |
|---|---|
| - Distéarate de sucrose commercialisé par la Société "STEARINERIE DUBOIS" | 2 % |
| - Stéarate de sorbitane oxyéthyléné à 4 moles d'oxyde d'éthylène commercialisé sous le nom de "TWEEN 61" par la Société ICI | 1,4 % |
| - Acide stéarique | 0,75 % |
| - Stearyl heptanoate commercialisé par la société DRAGOCO sous le nom de PCL solide | 5,5 % |
| - Vaseline codex | 2,1 % |
| - Huile d'avocat | 4,5 % |
| - Huile de jojoba | 4,1 % |
| - Huile de silicone volatile | 3,7 % |
| - Acétate de Vitamine E | 0,5 % |
| - D α tocophérol commercialisé par la Société HENKEL sous le nom de "COPHEROL 1300" | 0,3 % |

### Phase A2 :

| | |
|---|---|
| - Gomme de silicone commercialisée par la Société DOW CORNING sous le nom "Q₂-1403 Fluid" | 4 % |
| - Parfum | 0,3 % |
| - Propylparaben | 0,1 % |

### Phase B :

| | |
|---|---|
| - Glycérine | 5 % |
| - Méthyl paraben | 0,3 % |
| - Triéthanolamine | 0,4 % |
| - Eau déminéralisée | q.s.p. 100, % |

### Phase C :

| | |
|---|---|
| - Mélange de polymères carboxyvinyliques commercialisé sous le nom de "CARBOPOL 940" par la Société GOODRICH | 0,3 % |
| - Eau déminéralisée | 9,7 % |

La taille moyenne des globules d'huile de l'émulsion stabilisée est de 170 nm avec un indice de polydispersité de 0,09.

On obtient ainsi une crème blanche, lisse et brillante qui, à l'application, se révèle être douce, non grasse. Après plusieurs jours d'application, on constate une amélioration de l'état de sécheresse de la peau du visage.

### Exemple 2 : Crème de jour anti-âge pour le visage

### Phase A1 :

| | |
|---|---|
| - Distéarate de sucrose commercialisé par la Société STEARINERIE DUBOIS | 1,75 % |
| - Stéarate de sorbitane oxyéthyléné à 4 moles d'oxyde d'éthylène commercialisé par la Société ICI sous le nom "TWEEN 61" | 1,15 % |
| - Acide stéarique | 0,75 % |
| - Stearyl heptanoate | 4 % |
| - Vaseline codex | 1,5 % |
| - Huile d'avocat | 3,2 % |
| - Huile de jojoba | 3 % |
| - Huile de silicone volatile | 2,7 % |
| - Acétate de vitamine E | 1 % |
| - D α tocophérol naturel commercialisé par la Société HENKEL sous le nom "COPHEROL 1300" | 1 % |
| - Glycérides de Vitamine F | 3 % |
| - Palmitate de rétinol commercialisé par FLUKA dosé à 1500 Ul/mg | 0,5 % |

### Phase A2 :

| | |
|---|---|
| - Gomme de silicone commercialisé par DOW CORNING sous le nom "Q2-1403 Fluid" | 3 % |
| - Propylparaben | 0,2 % |
| - Parfum | 0,3 % |

### Phase B :

| | |
|---|---|
| - Glycérine | 3 % |
| - Hydroxyproline | 1 % |
| - D-panthenol | 1 % |
| - Triéthanolamine | 0,35 % |
| - Méthylparaben | 0,3 % |
| - Eau déminéralisée | q.s.p.100 % |

### Phase C :

| | |
|---|---|
| - Mélange de polymères carboxyvinyliques commercialisé sous le nom "CARBOPOL 940" par la Société GOODRICH | 0,4 % |
| - Eau déminéralisée | 9,6 % |

La taille moyenne des globules d'huile de l'émulsion stabilisée est de 190 nm avec un indice de polydispersité de 0,07.

On obtient une crème lisse, blanche, souple, fraîche et très confortable à l'application.

### Exemple 3 : Lait hydratant pour le corps

### Phase A1 :

| | |
|---|---|
| - Stéarate de sorbitane commercialisé par la Société ICI sous le nom "SPAN 60" | 1,5 % |
| - Stéarate de sorbitane oxyéthyléné à 4 moles d'oxyde d'éthylène commercialisé par la Société ICI sous le nom de "TWEEN 61" | 1 % |
| - Acide stéarique | 0,5 % |
| - Acide béhénique | 0,25 % |
| - Stearyl heptanoate | 3 % |
| - Vaseline codex | 1 % |
| - Huile de silicone volatile | 4 % |
| - Huile de jojoba | 2 % |
| - Acétate de vitamine E | 0,5 % |

### Phase A2 :

| | |
|---|---|
| - Gomme de silicone commercialisée par la Société DOW CORNING sous le nom "Q₂-1403 Fluid" | 2 % |
| - Propylparaben | 0,1 % |
| - Parfum | 0,3 % |

### Phase B :

| | |
|---|---|
| - Glycérine | 5 % |
| - Méthylparaben | 0,3 % |
| - Propylène glycol | 3 % |
| - Triéthanolamine | 0,25 % |
| - Eau déminéralisée | qsp 100 % |

### Phase C :

| | |
|---|---|
| - Mélange de polymères carboxyvinyliques commercialisé sous le nom "CARBOPOL 940" par la Société GOODRICH | 0,2 % |
| - Eau déminéralisée | 9,8 % |

La taille moyenne des globules d'huile de l'émulsion stabilisée est de 165 nm avec un indice de polydispersité de 0,09.

On obtient un lait fluide, frais et très couvrant.

### Exemple 4 : Crème de nuit aux liposomes non ioniques pour peaux stressées, et désorganisées

### Phase A1 :

| | |
|---|---|
| - Tristéarate de tétraglycéryle commercialisé par la Société NIKKOL sous le nom "TETRAGLYN 3 S" | 2 % |
| - Stéarate de sorbitane oxyéthyléné à 4 moles d'oxyde d'éthylène commercialisé par la Société ICI sous le nom de "TWEEN 61" | 1,4 % |
| - Acide stéarique | 1 % |
| - Stearyl heptanoate | 5,5 % |
| - Vaseline codex | 2,1 % |
| - Huile de macadamia | 4,5 % |
| - Huile d'amande d'abricot | 3,5 % |
| - Huile de silicone volatile | 3,7 % |
| - Acétate de Vitamine E | 1 % |
| - Glycérides de Vitamine F | 3 % |
| - D α tocophérol naturel commercialisé par HENKEL sous la référence "COPHEROL 1300" | 0,5 % |

### Phase A2 :

| | |
|---|---|
| - Gomme de silicone commercialisée par DOW CORNING sous le nom "Q₂-1403" | 4 % |
| - Propylparaben | 0,1 % |
| - Parfum | 0,3 % |

### Phase B :

| | |
|---|---|
| - Méthylparaben | 0,3 % |
| - Triéthanolamine | 0,4 % |
| - Eau déminéralisée | qsp 100 % |

### Phase C : Elle se décompose ici en deux phases C1 et C2

### Phase C1 : Phase vésiculaire

| | |
|---|---|
| - Tristéarate de tétraglycéryle commercialisé par NIKKOL sous le nom "TETRAGLYN 3 S" | 0,46 % |
| - Cholestérol | 0,46 % |
| - Sel monosodique de l'acide N-stéaroyl glutamique commercialisé sous le nom "ACYLGLUTAMATE HS 11" par la Société AJINOMOTO | 0,08 % |
| - Glycérine | 3,00 % |
| -Hydroxyproline | 1,00 % |
| - Eau déminéralisée | 5,00 % |

Cette phase est préparée de la façon suivante :
Les 3 lipides constituant la paroi lipidique des vésicules sont chauffés à la température de 115° nécessaire et suffisante pour en réaliser la co-fusion. On obtient ainsi un mélange liquide transparent que l'on refroidit à la température de 90° C.

On ajoute alors le reste de la phase aqueuse C1 pour réaliser l'hydratation du mélange lipidique à cette même température de 90° C par agitation lente pendant 60 minutes. On refroidit ce mélange à 60° C que l'on introduit alors dans l'homogénéisateur Soavi de type OBL réglé à la pression de 500 bars pour 3 passages successifs.

### Phase C2 :

| | |
|---|---|
| - Mélange de polymères carboxyvinyliques commercialisé sous le nom de "CARBOPOL 940" par la Société GOODRICH | 0,3 % |
| - Eau déminéralisée | 9,7 % |

La phase C1, refroidie à la température ambiante, est mélangée à la phase gélifiée C2 pour constituer la phase C.

La taille moyenne des globules d'huile de l'émulsion stabilisée est de 180 nm et l'indice de polydispersité est de 0,07.

On obtient une crème blanche, lisse et brillante de grand confort après application.

### Exemple 5 : Crème de jour aux liposomes pour peaux sensibles, stressées, désorganisées.

### Phase A1 :

| | |
|---|---|
| - Monostéarate de diglycéryle commercialisé par la Société NIKKOL sous la référence "DGMS" | 1,5 % |
| - Monostéarate polyoxyéthyléné 8-OE commercialisé par ICI sous le nom de Myrj 45 | 1 % |
| - Acide stéarique | 0,75 % |
| - Stearyl heptanoate | 4 % |
| - Vaseline codex | 1 % |
| - Silicone volatile | 3,2 % |
| - Huile de jojoba | 3 % |
| - Huile d'amande douce | 2,7 % |
| - Acétate de vitamine E | 0,5 % |
| - D α tocophérol naturel commercialisé par la Société HENKEL sous le nom "COPHEROL 1300" | 1 % |
| - Octylméthoxycinnamate commercialisé par la Société GIVAUDAN sous le nom "PARSOL MCX" | 2 % |
| - Butylméthoxydibenzoylméthane commercialisé par la Société GIVAUDAN sous le nom "PARSOL 1789" | 0,5 % |

### Phase A2 :

| | |
|---|---|
| - Gomme de silicone commercialisée par la Société DOW CORNING sous le nom "Q₂-1403 Fluid" | 3 % |
| - Conservateur | 0,1 % |
| - Parfum | 0,3 % |

### Phase B :

| | |
|---|---|
| - Conservateurs | 0,1 % |
| - Parfum | 0,3 % |
| - Triéthanolamine | 0,35 % |
| - Eau déminéralisée | qsp100 % |

### Phase C : Elle se compose de deux phases C1 et C2:

### Phase C1 : Phase vésiculaire

| | |
|---|---|
| - Mélange de phospholipides dans un mélange eau/alcool commercialisé sous le nom "NATIPIDE II" par la Société NATTERMAN PHOSPHOLIPID | 5 % |
| - Glycérine | 3 % |
| - Eau déminéralisée | 9 % |
| - Hydroxyproline | 1 % |

Cette phase est préparée en dispersant le NATIPIDE II à température ambiante à l'aide d'un barreau aimanté tournant à la vitesse de 300 t/mn pendant 30 mn dans le reste de la phase aqueuse.

### Phase C2 :

| | |
|---|---|
| - Polymère carboxyvinylique commercialisé par la Société SIGMA sous le nom "SYNTHALEN K" | 0,5 % |
| - Eau déminéralisée | 9,5 % |

La phase C1 est alors mélangée à la phase C2 pour constituer la phase C.

La taille moyenne des globules d'huile de l'émulsion stabilisée est de 180 nm et l'indice de polydispersité est de 0,08.

On obtient une crème blanche, lisse à texture fine, non grasse et très confortable.

### Exemple 6 : Crème de nuit restructurante pour peau rugueuse

### Phase A1 :

| | |
|---|---|
| - Monostéarate de diglyceryle commercialisé par la Société NIKKOL sous la référence DGMS | 2 % |
| - Monostéarate polyoxyéthyléné 10-0E commercialisé par NIKKOL sous le nom de Mys 10 | 1,35 % |
| - Acide stéarique | 1 % |
| - Stearyl heptanoate | 5,5 % |
| - Vaseline codex | 2 % |
| - Acétate de vitamine E | 3,4 % |
| - D α tocophérol naturel, commercialisé par la Société HENKEL sous le nom de "COPHEROL 1300" | 1 % |
| - Mélange d'huiles végétales stabilisé commercialisé par la Société NESTLE sous le nom "Huile Balance" | 3,4 % |
| - Mélange de Farnesol, acétate de farnesyle commercialisé par la Société INDUCHEM sous le nom " UNIBIOVIT B 33" | 1 % |
| - Palmitate de rétinol Fluka dosé à 1500 Ul/mg | 0,5 % |
| - Silicone volatile | 4,6 % |
| - Di tertiobutyl-3,5 hydroxy-4 benzylidène-3 camphre commercialisé par la Société CHIMEX sous le nom "MEXORYL SAD" | 0,5 % |

### Phase A2 :

| | |
|---|---|
| - Gomme de silicone commercialisée par la Société DOW CORNING sous le nom "Q₂-1403 Fluid" | 6 % |

### Phase B :

| | |
|---|---|
| - Conservateurs | 0,4 % |
| - Triéthanolamine | 0,4 % |
| - Glycérine | 3 % |
| - Hydroxylproline | 1 % |
| - D-panthenol | 1 % |
| - Eau déminéralisée | qsp 100 % |

### Phase C :

| | |
|---|---|
| - Mélange de polymères carboxyvinyliques commercialisé par la Société GOODRICH sous le nom "CARBOPOL 940" | 0,3 % |
| - Eau déminéralisée | 9,7 % |

La taille moyenne des globules d'huile de l'émulsion stabilisée est de 180 nm avec un indice de polydispersité de 0,07

On obtient une crème blanche, épaisse, riche mais non grasse à l'application et d'un grand confort.

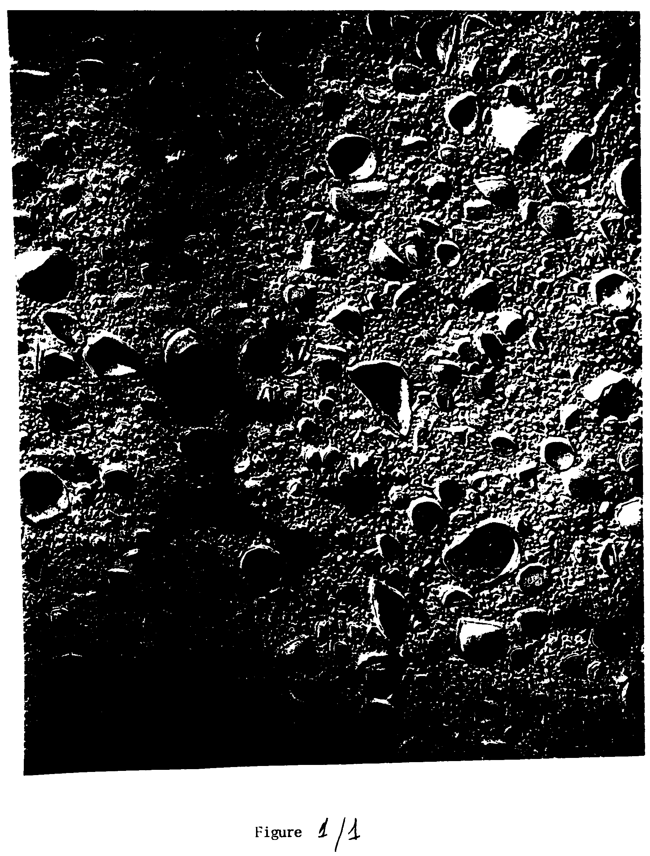

En plus du comptage effectué à l'aide du granulomètre à diffusion laser AMTECH BI 90 qui permet de déterminer la taille moyenne de la distribution en volume des globules huileux enrobés, soit 180 nm avec un indice de polydispersité de 0,07 on soumet cette crème à un examen en microscopie électronique par cryofracture. On peut ainsi constater sur le micrographe caractéristique, objet de la figure annexée (grossissement X 40 000 ; Echelle 1 mm (sur la photographie) = 25 nm (objet photographié), que les globules huileux sont unitaires, indépendants les uns des autres, enrobés par une couche majoritairement unilamellaire, parfois oligolamellaire et que la taille moyenne du plus grand nombre des globules huileux enrobés est d'environ 100 nm.

### Exemple 7 : Crème solaire de forte protection

### Phase A1 :

| | |
|---|---|
| - Distéarate de sucrose commercialisé par la Société STEARINERIE DUBOIS | 2 % |
| - Stéarate de sorbitane oxyéthyléné à 4 moles d'oxyde d'éthylène commercialisé par la Société ICI sous le nom "TWEEN 61" | 1,35 % |
| - Acide stéarique | 1 % |
| - Vaseline codex | 2,1 % |
| - Purcellin liquide | 5,5 % |
| - Octylméthoxycinnamate commercialisé par la Société GIVAUDAN ROURE sous le nom "Parsol MCX" | 7 % |
| - Butyl méthoxy dibenzoylméthane commercialisé par la Société GIVAUDAN ROURE sous le nom "Parsol 1789" | 4 % |
| - Silicone volatile | 3 % |
| - Huile de jojoba | 4 % |
| - Acétate de Vitamine E | 0,5 % |

### Phase A2 :

| | |
|---|---|
| - Gomme de silicone commercialisé par la Société DOW CORNING sous le nom "Q₂-1403 Fluid" | 4 % |
| - Conservateurs | 0,1 % |

### Phase B :

| | |
|---|---|
| - Conservateurs | 0,3 % |
| - EDTA disodique | 0,05 % |
| - Glycérine | 3 % |
| - Triéthanolamine | 0,4 % |
| - Eau déminéralisée | qsp 100 % |

### Phase C :

| | |
|---|---|
| - Polymère carboxyvinylique commercialisé par la Société SIGMA sous le nom "SYNTHALEN K" | 0,4 % |
| - Eau déminéralisée | 9,6 % |

La taille moyenne des globules d'huile de l'émulsion stabilisée est de 160 nm avec un indice de polydispersité de 0,07.

On obtient ainsi une crème épaisse blanche ayant un indice de protection de SPF 11 ayant une bonne rémanence au lavage à l'eau et une bonne persistance dans le temps.

### Exemple 8 : Lait parfumé pour le corps

### Phase A :

| | |
|---|---|
| - Monostéarate de diglycéryle commercialisé par la Société NIKKOL sous le nom DGMS | 1,5 % |
| - Stéarate de sorbitane oxyéthyléné à 4 moles d'oxyde d'éthylène commercialisé par la Société ICI sous le nom "TWEEN 61" | 1 % |
| - Acide stéarique | 0,75 % |
| - Stéaryl heptanoate | 2 % |
| - Huile de sésame | 6 % |
| - Huile de silicone volatile | 2 % |
| - Huile essentielle de bergamotte (exempte de Bergaptène) | 8 % |

### Phase B :

| | |
|---|---|
| - Gomme de silicone commercialisée par la Société DOW CORNING sous le nom "Q₂-1403 Fluid" | 2 % |
| - Conservateur | 0,1 % |

### Phase C :

| | |
|---|---|
| - Glycérine | 3 % |
| - Propylène glycol | 5 % |
| - Conservateurs | 0,3 % |
| - Triéthanolamine | 0,3 % |
| - Eau déminéralisée | qsp100 % |

La taille moyenne des globules d'huile de l'émulsion stabilisée est de 160 nm avec un indice de polydispersité de 0,06

On obtient un lait blanc, très fluide, pulvérisable à l'aide d'un flacon-pompe. Propriétés parfumantes rémanentes dans le temps.

### Exemple 9 : Crème de jour pour peaux mixtes aux liposomes non-ioniques

### Phase A1 :

| | |
|---|---|
| - Distéarate de diglycéryl commercialisé par la Société NIHON EMULSION sous le nom "EMALEX DSG2" | 1,5 % |
| - Distéarate de méthylglucose polyoxyéthyléné 20-OE commercialisé par la société AMERCHOL sous le nom de Glucam E 20 distearate | 1 % |
| - Acide stéarique | 0,25 % |
| - Acide palmitique | 0,5 % |
| - Stéaryl heptanoate | 4 % |
| -Vaseline codex | 1,5 % |
| - Huile de jojoba | 3 % |
| - Huile de macadamia | 3,2 % |
| - Huile de silicone volatile | 3 % |
| - Acétate de vitamine E | 0,5 % |
| - D α tocophérol naturel commercialisé par la Société HENKEL sous le nom "COPHEROL 1300" | 0,3 % |
| - Glycéride de vitamine F | 1 % |

### Phase A2 :

| | |
|---|---|
| - Gomme de silicone commercialisée par la Société DOW CORNING sous le nom "Q₂-1403 Fluid" | 3 % |
| - Conservateurs | 0,1 % |
| - Parfum | 0,3 % |

### Phase B :

| | |
|---|---|
| - Propylène glycol | 3 % |
| - Conservateurs | 0,4 % |
| - Triéthanolamine | 0,3 % |
| - Eau déminéralisée | qsp 100 % |

### Phase C :

Cette phase se décompose en deux phases : C1 et C2. On opère comme dans l'exemple 4.

### Phase C1 : Phase vésiculaire

| | |
|---|---|
| - Monodipalmitostéarate de saccharose commercialisé par la Société GRILLO sous le nom "GRILLOTEN PSE 141G" | 0,46 % |
| - Cholestérol | 0,46 % |
| - Sel monosodique de l'acide N-stéaroyl glutamique commercialisé par la société AJINOMOTO sous le nom "ACYLGLUTAMATE HS11" | 0,08 % |
| - Glycérine | 3 % |
| - Hydroxyproline | 1 % |
| - Eau déminéralisée | 5 % |

### Phase C2 :

| | |
|---|---|
| - Polymère carboxyvinylique commercialisé par la Société SIGMA sous le nom "SYNTHALEN K" | 0,3 % |
| - Eau déminéralisée | 9,7 % |

La taille moyenne des globules d'huile dans l'émulsion stabilisée est de 180 nm avec un indice de polydispersité de 0,07.

On obtient une crème blanche, fine et brillante, très confortable à l'application, non grasse et évanescente.

### Exemple 10 : Crème de jour protectrice pour peaux à tendance sèche

### Phase A :

| | |
|---|---|
| - Distéarate de sucrose commercialisé par la Société STEARINERIE DUBOIS | 1,75 % |
| - Stéarate de sorbitane oxyéthyléné à 4 moles d'oxyde d'éthylène commercialisé par la Société ICI sous le nom "TWEEN 61" | 1,15 % |
| - Acide stéarique | 0,85 % |
| - Tétrastéarate de pentaerythritol commercialisé par la Société AKZO sous le nom "KESSCO PTS" | 1 % |
| - Mélange de mono et dibéhénate de glycéryle commercialisé par la Société GATTEFOSSE sous le nom de "BEHENATE de glycéryle WL 251 | 1 % |
| - Vaseline codex | 1,5 % |
| - Stéaryl heptanoate | 5 % |
| - Huile de jojoba | 3,5 % |
| - Huile d'amande douce | 3 % |
| - Huile de silicone volatile | 3 % |
| - Glycéride de Vitamine F | 1 % |
| - Acétate de vitamine E | 0,5 % |
| - Phytantriol commercialisé par la Société HOFFMANN LAROCHE | 1 % |

### Phase A2 :

| | |
|---|---|
| - Gomme de silicone commercialisé par la Société DOW CORNING sous le nom "Q₂-1403 Fluid" | 3 % |
| - Conservateur | 0,1 % |
| -:Parfum | 0,3 % |

### Phase B :

| | |
|---|---|
| - Conservateurs | 0,3 % |
| - Glycérine | 3 % |
| - Hydroxyproline | 1 % |
| - Lysine | 0,4 % |
| - Eau déminéralisée | qsp 100 % |

### Phase C :

| | |
|---|---|
| - Polymère carboxyvinylique commercialisé par la Société SIGMA sous le nom "SYNTHALEN K" | 0,4 % |
| - Eau déminéralisée | 9,6 % |

La taille moyenne des globules d'huile de l'émulsion stabilisée est de 200 nm avec un indice de polydispersité de 0,05.

On obtient une crème blance, lisse qui présente de bonnes qualités de protection notamment pour les peaux sèches en période hivernale.

### Exemple 11 : Crème teintée destinée aux peaux claires

### Phase A1 :

| | |
|---|---|
| - Distéarate de sucrose commercialisé par la Société STEARINERIE DUBOIS | 2 % |
| - Stéarate de sorbitane oxyéthyléné à 4 moles d'oxyde d'éthylène commercialisé par la Société ICI sous le nom "TWEEN 61" | 1,35 % |
| - Acide stéarique | 1 % |
| - Stearyl heptanoate | 5,5 % |
| - Vaseline codex | 2,1 % |
| - Huile de silicone volatile | 6 % |
| - Huile d'avocat | 4 % |
| - Huile de jojoba | 4 % |
| - Acétate de DL α tocophérol | 0,5 % |
| - Ethoxyquine | 0,03 % |

### Phase A2 :

| | |
|---|---|
| - Gomme de silicone commercialisé par la Société DOW CORNING sous le nom "Q₂-1403 Fluid" | 4 % |
| - Propylparaben | 0,1 % |
| - Parfum | 0,3 % |

### Phase B :

| | |
|---|---|
| - Méthyl paraben | 0,1 % |
| - Germal II | 0,3 % |
| - Dequest 2046 | 0,05 % |
| - Glycérine | 3 % |
| - Triéthanolamine | 0,3 % |
| - Eau déminéralisée | 45 % |

### Phase C :

| | |
|---|---|
| - Saponite commercialisée par la Société VANDERBILT sous le nom "VEEGUM" | 0,35 % |
| - Oxydes de fer jaunes | 0,77 % |
| - Oxydes de fer bruns | 0,77 % |
| - Oxydes de fer noirs | 0,35 % |
| - Dioxydes de titane | 5,11 % |
| - Gomme de xanthane commercialisée par la Société KELCO sous le nom "KELTROL T" | 0,2 % |
| - Eau déminéralisée | qsp 100 % |

La taille moyenne des globules d'huile enrobés est de 180 nm avec un indice de polydispersité de 0,08.

On obtient ainsi une crème teintée, lisse couvrant bien uniformément les imperfections du teint du visage.

### Exemple 12 : Crème de jour antisolaire à fort indice de protection

### Phase A1 :

| | |
|---|---|
| - Distéarate de sucrose commercialisé par la Société STEARINERIE DUBOIS | 1,5 % |
| - Stéarate de sorbitane oxyéthyléné à 4 moles d'oxyde d'éthylène commercialisé par la Société ICI sous le nom "TWEEN 61" | 1 % |
| - Acide stéarique | 0,5 % |
| - Acide arachidique | 0,25 % |
| - Stearyl heptanoate | 5,5 % |
| - Vaseline codex | 2,1 % |
| - Huile d'avocat | 4 % |
| - Huile de jojoba | 4 % |
| - Acétate de DL α tocophérol | 0,5 % |
| - Octyl méthoxycinnamate commercialisé par la Société GIVAUDAN ROURE sous le nom "PARSOL MCX" | 2 % |
| - Butylméthoxydibenzoylméthane commercialisé par la Société GIVAUDAN ROURE sous le nom de "PARSOL 1789" | 0,5 % |
| - Ethoxyquine | 0,03 % |

### Phase A2 :

| | |
|---|---|
| - Dioxyde de titane nanométrique commercialisé par la Société TAYCA sous le nom "MT 100 T" | 10 % |
| - Dodécaméthyl cyclo hexa siloxane commercialisé par la Société HERCULES | 10 % |
| - Gomme de silicone commercialisée par la Société DOW CORNING sous le nom "Q₂-1403 Fluid" | 4 % |

### Phase B :

| | |
|---|---|
| - Conservateurs | 0,4 % |
| - Sequestrant | 0,05 % |
| - Glycérine | 3,0 % |
| - Triéthanolamine | 0,4 % |
| - Eau déminéralisée | 42,67 % |

### Phase C :

| | |
|---|---|
| - Mélange de polymères carboxyvinyliques commercialisé par la Société GOODRICH sous le nom "CARBOPOL 940" | 0,3 % |
| - Eau déminéralisée | qsp 100 % |

La taille moyenne des globules d'huile enrobés est de 170 nm avec un indice de polydispersité de 0,12

On obtient ainsi une crème blanche, lisse, mate, épaisse (46 poises).

### Exemple 13 : Crème de jour vitaminée pour le soin du visage

### Phase A1 :

| | |
|---|---|
| - Distéarate de sucrose commercialisé par la Société STEARINERIE DUBOIS | 1,75 % |
| - Stéarate de sorbitane oxyéthyléné à 4 moles d'oxyde d'éthylène commercialisé par la Société ICI sous le nom "TWEEN 61" | 1,15 % |
| - Acide stéarique | 0,75 % |
| - Acétate de D α tocophérol | 1 % |
| - Glycérides de vitamine F | 2 % |
| - Palmitate de rétinol dosé à 1500 Ul/mg commercialisé par la société FLUKA | 0,5 % |
| - Palmitate d'ascorbyle | 0,5 % |
| - Mélange de Farnesol, acétate de farnesyle commercialisé par la société INDUCHEM sous le nom "UNIBIOVIT B 33" | 2 % |
| - Huile de cassis | 3 % |
| - Ethoxyquine | 0,03 % |

### Phase B :

| | |
|---|---|
| - Glycérine | 3 % |
| - Hydroxyproline | 1 % |
| - D panthénol | 1 % |
| - Lysine | 0,4 % |
| - Eau déminéralisée | 50 % |

### Phase C :

| | |
|---|---|
| - Polymère carboxyvinylique commercialisé par la Société SIGMA sous le nom "SYNTHALEN K" | 0,4 % |
| - Eau déminéralisée | qsp 100 % |

On obtient ainsi une crème blanche, lisse, fluide qui présente un effet stimulant pour les peaux ternes, fatiguées.

### Exemple 14 : Crème de jour antisolaire

### Phase A1 :

| | |
|---|---|
| - Distéarate de sucrose commercialisé par la Société STEARINERIE DUBOIS | 2 % |
| - Stéarate de sorbitane oxyéthyléné à 4 moles d'oxyde d'éthylène commercialisé par la Société ICI sous le nom "TWEEN 61" | 1,35 % |
| - Acide stéarique | 1 % |
| - Stéaryl heptanoate | 5,5 % |
| - Vaseline codex | 2,1 % |
| - Propylparaben | 0,08 % |
| - Huile de silicone volatile | 3,7 % |
| - Huile de jojoba | 4,5 % |
| - Huile d'avocat | 4,1 % |
| - Acétate de D α tocophérol | 0,3 % |
| - 2-benzotriazol-2-yl-4-méthyl-6-[3-[1,3,3,3-tétraméthyl-1-[(tri-méthylsilyl)oxy]disiloxanyl]2-méthyl-propyl] phénol | 7 % |

### Phase A2 :

| | |
|---|---|
| - Gomme de silicone commercialisée par la Société DOW CORNING sous le nom "Q₂-1403 Fluid" | 3 % |

### Phase B :

| | |
|---|---|
| - Conservateurs | 0,4 % |
| - Glycérine | 3 % |
| - Triéthanolamine | 0,4 % |
| - Eau déminéralisée | 51 % |

### Phase C :

| | |
|---|---|
| - Mélange de polymères carboxyvinyliques commercialisé par la Société GOODRICH sous le nom "CARBOPOL 940" | 0,3 % |
| - Eau déminéralisée | qsp 100 % |

La taille moyenne des globules d'huile est de 200 nm avec un indice de polydispersité de 0,09.

On obtient une crème blanche, lisse, brillante et épaisse (60 poises).

### Exemple 15 : Crème de jour

### Phase A :

| | |
|---|---|
| - Distéarate de diglycéryle commercialisé par la Société NIHON EMULSION sous la référence Emalex DSG2 | 1,5 % |
| - Distéarate de méthylglucose polyoxyéthyléné 20-0E commercialisé par la société AMERCHOL sous le nom de Glucam E20 distearate | 1 % |
| - Acide stéarique | 0,75 % |
| - Stearyl heptanoate | 2 % |
| - Perfluorodécaline commercialisée par la société | |
| FLUOROCHEM Limited | 6 % |
| - Huile de sésame | 6 % |
| - Huile de silicone volatile | 3 % |
| - Huile de jojoba | 3,5 % |
| - Propylparaben | 0,1 % |
| - D α tocophérol naturel, commercialisé par la Société HENKEL sous le nom de "COPHEROL 1300" | 0,3 % |

### Phase B :

| | |
|---|---|
| - Conservateurs | 0,4 % |
| - Glycérine | 3 % |
| - Triéthanolamine | 0,3 % |
| - Eau déminéralisée | qsp 60 % |

### Phase C :

| | |
|---|---|
| - Mélange de polymères carboxyvinyliques commercialisé par la Société GOODRICH sous le nom "CARBOPOL 940" | 0,3 % |
| - Eau déminéralisée | qsp 100 % |

On obtient une crème très fluide, très fine, blanche et brillante.

## Revendications

1. Composition cosmétique ou dermatologique constituée par une émulsion du type huile dans eau formée de globules huileux pourvus chacun d'un enrobage cristal liquide lamellaire et dispersés dans une phase aqueuse, caractérisée en ce que chaque globule huileux contenant au moins un composé lipophile actif cosmétique ou dermatologique est unitairement enrobé d'une couche monolamellaire ou oligolamellaire obtenue à partir d'au moins un agent tensioactif lipophile, d'au moins un agent tensioactif hydrophile et d'au moins un acide gras, les globules huileux enrobés ayant un diamètre moyen inférieur à 500 nanomètres, et en ce que la phase aqueuse contient à l'état dissous un agent basique.

2. Composition selon la revendication 1, caractérisée en ce que les globules huileux ont un diamètre moyen inférieur à 200 nanomètres.

3. Composition selon l'une quelconque des revendications précédentes, caractérisée en ce que l'agent tensioactif lipophile, l'agent tensioactif hydrophile et l'acide gras comportent chacun au moins une chaine grasse saturée ayant plus de 12 atomes de carbone environ, de préférence entre 16 et 22.

4. Composition selon l'une quelconque des revendications précédentes, caractérisée en ce que l'agent tensioactif lipophile présente un HLB (balance hydrophile-lipophile) compris entre 2 et 5.

5. Composition selon la revendication 4, caractérisée en ce que l'agent tensioactif lipophile présentant un HLB compris entre 2 et 5 est choisi parmi le groupe comprenant le distéarate de sucrose, le distéarate de diglycéryle, le tristéarate de tétraglycéryle, le décastéarate de décaglycéryle, le monostéarate de diglycérol, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthyléné 2 OE, (comprenant 2 motifs d'oxyde d'éthylène), le mono et dibéhénate de glycéryle, le tétrastéarate de pentaérythrytol.

6. Composition selon l'une quelconque des revendications précédentes, caractérisée en ce que l'agent tensioactif hydrophile présente un HLB compris entre 8 et 12.

7. Composition selon la revendication 6, caractérisée en ce que l'agent tensioactif hydrophile présentant un HLB compris entre 8 et 12 est choisi parmi le groupe comprenant le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthyléné 12 OE, le distéarate de méthylglucose polyoxyéthyléné 20 OE.

8. Composition selon l'une quelconque des revendications précédentes, caractérisée en ce que l'acide gras est choisi parmi le groupe comprenant l'acide palmitique, l'acide stéarique, l'acide arachidique et l'acide béhénique.

9. Composition selon l'une quelconque des revendications précédentes, caractérisée en ce que l'agent tensioactif lipophile, l'agent tensioactif hydrophile et l'acide gras sont présents en une quantité comprise entre 2 et 6 % en poids par rapport au poids total de la composition et de préférence entre 3 et 4 %.

10. Composition selon l'une quelconque des revendications précédentes, caractérisée en ce que l'agent tensioactif lipophile, l'agent tensioactif hydrophile et l'acide gras varient de préférence dans les fourchettes respectives de 35-55%, 25-40% et 15-35% en poids par rapport à leur poids total.

11. Composition selon l'une quelconque des revendications précédentes, caractérisée en ce que les globules huileux enrobés représentent de 5 à 50% en poids par rapport au poids total de la composition, de préférence de 10 à 40%.

12. Composition selon l'une quelconque des revendications précédentes, caractérisée en ce que le rapport pondéral des globules huileux aux tensioactifs constitutifs de l'enrobage est compris entre 2 et 13, de préférence égal à 7 environ.

13. Composition selon l'une quelconque des revendications précédentes, caractérisée en ce que l'agent basique est dissous dans la phase aqueuse en une quantité au moins égale à la quantité nécessaire à la neutralisation de l'acide gras.

14. Composition selon l'une quelconque des revendications précédentes, caractérisé en ce que l'agent basique est choisi parmi le groupe comprenant la soude, la triéthanolamine, la lysine et l'arginine.

15. Composition selon l'une quelconque des revendications précédentes, caractérisée en ce que la phase aqueuse contient en outre un ou plusieurs composés hydrophiles cosmétiquement ou dermatologiquement actifs, libres ou encapsulés.

16. Composition selon la revendication 15, caractérisée en ce que le composé hydrophile cosmétiquement ou dermatologiquement actif est encapsulé dans un vésicule lipidique ionique et/ou non ionique ou dans une nanoparticule, nanosphère, nanoéponge ou nanocapsule.

17. Composition selon l'une quelconque des revendications précédentes, caractérisée en ce que les globules huileux contiennent au moins une substance grasse ou lipophile ayant une activité pour le soin de la peau.

18. Composition selon la revendication 17, caractérisée en ce que la substance grasse ou lipophile est choisi parmi les agents antioxydants, anti radicaux libres, hydratants, mélanorégulateurs, accélérateurs de bronzage, dépigmentants, de coloration de la peau, liporégulateurs, amincissants, anti-acnéiques, antiseborrheiques, anti-vieillissement, anti-rides, anti-UV, kératolytiques, anti-inflammatoires, rafraichissants, cicatrisants, protecteurs vasculaires, antibactériens, antifungiques, antiperspirants, déodorants, conditionneurs de la peau, immunomodulateurs, nourrissants et les huiles essentielles et les parfums.

19. Composition selon l'une quelconque des revendications 1 à 16, caractérisée en ce les globules huileux contiennent au moins une substance grasse ou lipophile ayant une activité pour le soin du cheveu.

20. Composition selon la revendication 19, caractérisée en ce que la substance grasse ou lipophile est choisi parmi les agents mélanorégulateurs, liporégulateurs, antiséborrhéiques, anti-vieillissement, anti-UV, kératolytiques, antibactériens, antifungiques, antipelliculaires, anti-chute des cheveux, colorants capillaires, décolorants capillaires, réducteurs pour permanente, conditionneurs des cheveux et nourrissants.

21. Composition selon la revendication 18 ou 20, caractérisée en ce que la substance grasse ou lipophile est choisie parmi le groupe comprenant les composés suivants: D α tocophérol, DL α tocophérol, acétate de D α tocophérol, acétate DL αtocophérol, palmitate d'ascorbyle, glycérides de vitamine F, vitamines D, vitamine D₂, vitamine D₃, rétinol, esters de rétinol, β carotène, D panthénol, farnesol, acétate de farnesyle, huiles de jojoba, de cassis riches en acides gras essentiels, acide n octanoyl-5 salicylique, acide salicylique, alkylesters d'αhydroxyacides tels que acide citrique, acide lactique, acide glycolique, acide asiatique, acide madécassique, asiaticoside, extrait total de Centella asiatica, acide β glycyrrhétinique, α bisabolol, 2 oleoylamino-1,3 octadécane, phytanetriol, sphingomyéline de lait, phospholipides d'origine marine riches en acides gras essentiels polyinsaturés, éthoxyquine, extrait de romarin, extrait de mélisse, quercetine, extrait de microalgues séchées, huile essentielle de bergamotte, octylmethoxycinnamate, butylméthoxydibenzoyl-méthane, octyl triazone, oxydes de fer jaune, brun, noir, rouge, oxydes de titane sous forme micrométrique, nanométrique ou enrobée, di tertiobutyl-3,5 hydroxy-4 benzylidène 3 camphre, 2-benzotriazol-2-yl-4-méthyl-6-[3-[1,3,3,3-tétraméthyl-1-[(triméthylsilyl)oxy]disiloxanyl]-2-méthyl-propyl] phénol, huile perfluorée, huile de maïs hyperoxygénée.

22. Composition selon l'une quelconque des revendications précédentes, caractérisée en ce que le rapport huile/eau est au plus égal à 1.

23. Procédé de préparation d'une composition selon l'une quelconque des revendications précédentes, caractérisé en ce que dans une première étape on mélange sous agitation la phase huileuse comprenant le tensioactif lipophile, le tensioactif hydrophile, l'acide gras, le composé actif au plan cosmétique ou dermatologique et la phase aqueuse comprenant l'agent neutralisant et dans une deuxième étape on soumet le mélange obtenu à une homogénéisation basée sur le principe de la cavitation.

24. Procédé selon la revendication 23, caractérisée en ce que l'homogénéisation basée sur le principe de la cavitation est obtenue soit à l'aide de hautes pressions comprises entre 200 et 1000 bars, soit à l'aide d'ultrasons, soit à l'aide d'homogénéisateurs équipés d'une tête rotor-stator.

25. Procédé selon l'une des revendications 23 et 24, caractérisée en ce que l'on introduit au moins un composé hydrophile cosmétiquement ou dermatologiquement actif à l'état libre dans la première étape.

26. Procédé selon l'une des revendications 23 et 25, caractérisée en ce que l'on mélange au moins un composé hydrophile cosmétiquement ou dermatologiquement actif à l'état encapsulé dans une troisième étape.

27. Emulsion du type huile dans eau formée de globules huileux pourvus chacun d'un enrobage cristal liquide lamellaire et dispersés dans une phase aqueuse, caractérisée en ce que chaque globule huileux est unitairement enrobé d'une couche monolamellaire ou oligolamellaire obtenue à partir d'au moins un agent tensioactif lipophile, d'au moins un agent tensioactif hydrophile et d'au moins un acide gras, les globules huileux enrobés ayant un diamètre moyen inférieur à 500 nanomètres, et en ce que la phase aqueuse contient à l'état dissous un agent basique.

28. Emulsion selon la revendication 27, caractérisée en ce que le rapport huile/eau est au plus égal à 1.

## Claims

1. Cosmetic or dermatological composition consisting of an emulsion of oil-in-water type formed of oily globules which are provided with a lamellar liquid crystal coating and are dispersed in an aqueous phase, characterized in that each oily globule containing at least one lipophilic compound which is cosmetically or dermatologically active is individually coated with a monolamellar or oligolamellar layer obtained from at least one lipophilic surface-active agent, from at least one hydrophilic surface-active agent and from at least one fatty acid, the coated oily globules having a mean diameter of less than 500 nanometres, and in that the aqueous phase contains a basic agent in the dissolved state.

2. Composition according to Claim 1, characterized in that the oily globules have a mean diameter of less than 200 nanometres.

3. Composition according to either of the preceding claims, characterized in that the lipophilic surface-active agent, the hydrophilic surface-active agent and the fatty acid each contain at least one saturated fatty chain having more than approximately 12 carbon atoms, preferably between 16 and 22.

4. Composition according to any one of the preceding claims, characterized in that the lipophilic surface-active agent has an HLB (hydrophilic-lipophilic balance) between 2 and 5.

5. Composition according to Claim 4, characterized in that the lipophilic surface-active agent having an HLB between 2 and 5 is chosen from the group comprising sucrose distearate, diglyceryl distearate, tetraglyceryl tristearate, decaglyceryl decastearate, diglyceryl monostearate, hexaglyceryl tristearate, decaglyceryl pentastearate, sorbitan monostearate, sorbitan tristearate, diethylene glycol monostearate, the ester of glycerol and palmitic and stearic acids, polyoxyethylenated monostearate 2 EO (containing 2 ethylene oxide units), glyceryl mono- and dibehenate and pentaerythritol tetrastearate.

6. Composition according to any one of the preceding claims, characterized in that the hydrophilic surface-active agent has an HLB between 8 and 12.

7. Composition according to Claim 6, characterized in that the hydrophilic surface-active agent having an HLB between 8 and 12 is chosen from the group comprising polyoxyethylenated sorbitan monostearate 4 EO, polyoxyethylenated sorbitan tristearate 20 EO, polyoxyethylenated monostearate 8 EO, hexaglyceryl monostearate, polyoxyethylenated monostearate 10 EO, polyoxyethylenated distearate 12 EO and polyoxyethylenated methylglucose distearate 20 EO.

8. Composition according to any one of the preceding claims, characterized in that the fatty acid is chosen from the group comprising palmitic acid, stearic acid, arachidic acid and behenic acid.

9. Composition according to any one of the preceding claims, characterized in that the lipophilic surface-active agent, the hydrophilic surface-active agent and the fatty acid are present in an amount between 2 and 6% by weight relative to the total weight of the composition and preferably between 3 and 4%.

10. Composition according to any one of the preceding claims, characterized in that the lipophilic surface-active agent, the hydrophilic surface-active agent and the fatty acid preferably vary within respective ranges of 35-55%, 25-40% and 15-35% by weight relative to their total weight.

11. Composition according to any one of the preceding claims, characterized in that the coated oily globules represent from 5 to 50% by weight relative to the total weight of the composition, preferably from 10 to 40%.

12. Composition according to any one of the preceding claims, characterized in that the weight ratio of the oily globules to the surfactants constituting the coating is between 2 and 13, preferably approximately equal to 7.

13. Composition according to any one of the preceding claims, characterized in that the basic agent is dissolved in the aqueous phase in an amount at least equal to the amount required to neutralize the fatty acid.

14. Composition according to any one of the preceding claims, characterized in that the basic agent is chosen from the group comprising sodium hydroxide, triethanolamine, lysine and arginine.

15. Composition according to any one of the preceding claims, characterized in that the aqueous phase additionally contains one or more free or encapsulated, cosmetically or dermatologically active hydrophilic compounds.

16. Composition according to Claim 15, characterized in that the cosmetically or dermatologically active hydrophilic compound is encapsulated in an ionic and/or nonionic lipid vesicle or in a nanoparticle, nanosphere, nanosponge or nanocapsule.

17. Composition according to any one of the preceding claims, characterized in that the oily globules contain at least one fatty or lipophilic substance having a skin-care activity.

18. Composition according to Claim 17, characterized in that the fatty or lipophilic substance is chosen from antioxidants, free radical scavengers, moisturizing agents, melanoregulators, tanning accelerators, depigmenting agents, skin-colouring agents, liporegulators, thinning agents, anti-acne agents, antiseborrhoeic agents, anti-ageing agents, anti-wrinkle agents, anti-UV agents, keratolytic agents, antiinflammatory agents, refreshing agents, cicatrizing agents, vascular protectors, antibacterial agents, antifungal agents, antiperspirants, deodorants, skin conditioners, immunomodulators, nutrients and essential oils and perfumes.

19. Composition according to any one of Claims 1 to 16, characterized in that the oily globules contain at least one fatty or lipophilic substance having a hair-care activity.

20. Composition according to Claim 19, characterized in that the fatty or lipophilic substance is chosen from melanoregulators, liporegulators, antiseborrhoeic agents, anti-ageing agents, anti-UV agents, keratolytic agents, antibacterial agents, antifungal agents, anti-dandruff agents, agents for combating hair loss, hair dyes, hair bleaches, reducing agents for permanent waves, hair conditioners and nutrients.

21. Composition according to Claim 18 or 20, characterized in that the fatty or lipophilic substance is chosen from the group comprising the following compounds: D-α-tocopherol, DL-α-tocopherol, D-α-tocopherol acetate, DL-α-tocopherol acetate, ascorbyl palmitate, glycerides of vitamin F, vitamin D, vitamin D₂, vitamin D₃, retinol, retinol esters, retinol palmitate, retinol propionate, β-carotene, D-panthenol, farnesol, farnesyl acetate, oils of jojoba and of blackcurrant rich in essential fatty acids, 5-n-octanoylsalicylic acid, salicylic acid, alkyl esters of α-hydroxy acids such as citric acid, lactic acid, glycolic acid, asiatic acid and madecassic acid, asiaticoside, whole extract of Centella asiatica, β-glycyrrhetinic acid, α-bisabolol, 2-oleoylamino-1, 3-octadecane, phytanetriol, sphingomyelin from milk, phospholipids of marine origin which are rich in polyunsaturated essential acids, ethoxyquine, extract of romarin, extract of balm, quercetin, extract of dried microalgae, essential oil of bergamot, octyl methoxycinnamate, butylmethoxy-dibenzoylmethane, octyl triazone, yellow, brown, black and red iron oxides, titanium oxides which may be provided in micrometric or nanometric form or in coated form, 3,5-di-tert-butyl-4-hydroxybenzylidene-3-camphor, 2-benzotriazole-2-yl-4-methyl-6-[3-[1, 3, 3, 3-tetramethyl-1-[(trimethylsilyl) oxy]disiloxanyl]-2-methyl propyl]phenol, perfluoronated oil, and hyperoxygenated corn oil.

22. The composition according to any one of the preceding claims, characterized in that the oil/water ratio is not greater than 1.

23. Process for the preparation of a composition according to any one of the preceding claims, characterized in that, in a first step, the oily phase comprising the lipophilic surfactant, the hydrophilic surfactant, the fatty acid, the cosmetically or dermatologically active compound and the aqueous phase comprising the neutralizing agent are mixed with stirring and, in a second step, the mixture obtained is subjected to a homogenization based on the principle of cavitation.

24. Process according to Claim 23, characterized in that the homogenization based on the principle of cavitation is obtained either using high pressures between 200 and 1000 bar or using ultrasound or using homogenizers equipped with a rotor-stator head.

25. Process according to either of Claims 23 and 24, characterized in that at least one cosmetically or dermatologically active hydrophilic compound in the free state is introduced in the first step.

26. Process according to either of Claims 23 to 25, characterized in that at least one cosmetically or dermatologically active hydrophilic compound in the encapsulated state is mixed in a third step.

27. An oil-in-water type emulsion which comprises oily globules each coated with a coating of lamellar liquid crystal and dispersed in an aqueous phase, characterized in that each oily globule is individually coated with a monolamellar or oligolamellar layer obtained from at least one lipophilic surface-active agent, at least one hydrophilic surface-active agent and at least one fatty acid, the coated globules having a mean diameter of less than 500 nanometers and in that the aqueous phase contains a basic agent in a dissolved state.

28. The emulsion according to Claim 27, wherein the oil/water ratio is not greater than 1.

## Patentansprüche

1. Kosmetische oder dermatologische Zusammensetzung, die aus einer Öl-in-Wasser-Emulsion besteht, die aus Ölkügelchen gebildet ist, die jeweils einzeln mit einer lamellaren flüssigkristallinen Umhüllung versehen sind und die in einer wäßrigen Phase dispergiert sind, dadurch **gekennzeichnet,** daß
jedes Ölkügelchen, das mindestens einen kosmetischen oder dermatologischen lipophilen Wirkstoff enthält, einheitlich mit einer monolamellaren oder oligolamellaren Schicht umhüllt ist, die aus mindestens einem lipophilen grenzflächenaktiven Stoff, mindestens einem hydrophilen grenzflächenaktiven Stoff und mindestens einer Fettsäure erhalten wurde, wobei die umhüllten Ölkügelchen einen mittleren Durchmesser unter 500 nm aufweisen, und daß die wäßrige Phase ein basisches Mittel in gelöster Form enthält.

2. Zusammensetzung nach Anspruch 1,
dadurch gekennzeichnet, daß
die Ölkügelchen einen mittleren Durchmesser unter 200 nm aufweisen.

3. Zusammensetzung nach einem der vorhergehenden Ansprüche,
dadurch gekennzeichnet, daß
der lipophile grenzflächenaktive Stoff, der hydrophile grenzflächenaktive Stoff und die Fettsäure jeweils mindestens eine gesättigte Fettsäurekette mit mehr als etwa 12 Kohlenstoffatomen, vorzugsweise 16 bis 22 Kohlenstoffatomen, enthalten.

4. Zusammensetzung nach einem der vorhergehenden Ansprüche,
dadurch gekennzeichnet, daß
der liphophile grenzflächenaktive Stoff einen HLB-Wert (Hydrophile-lipophile Balance) von 2 bis 5 aufweist.

5. Zusammensetzung nach Anspruch 4,
dadurch gekennzeichnet, daß
der lipophile, einen HLB-Wert von 2 bis 5 aufweisende grenzflächenaktive Stoff unter Saccharosedistearat, Diglyceryldistearat, Tetraglyceryltristearat, Decaglyceryldecastearat, Diglycerylmonostearat, Hexaglyceryltristearat, Decaglycerylpentastearat, Sorbitanmonostearat, Sorbitantristearat, Diethylenglykolmonostearat, dem Ester aus Glycerin und Palmitin- und Stearinsäure, polyethoxyliertem Monostearat 2 EO (enthält 2 Ethylenoxid-Einheiten), Glycerylmonobehenat, Glyceryldibehenat und Pentaerythrittetrastearat ausgewählt ist.

6. Zusammensetzung nach einem der vorhergehenden Ansprüche,
dadurch gekennzeichnet, daß
der hydrophile grenzflächenaktive Stoff einen HLB-Wert von 8 bis 12 aufweist.

7. Zusammensetzung nach Anspruch 6,
dadurch gekennzeichnet, daß
der einen HLB-Wert von 8 bis 12 aufweisende hydrophile grenzflächenaktive Stoff unter polyethoxyliertem Sorbitanmonostearat 4 EO, polyethoxyliertem Sorbitantristearat 20 EO, polyethoxyliertem Monostearat 8 EO, Hexaglycerylmonostearat, polyethoxyliertem Monostearat 10 EO, polyethoxyliertem Distearat 12 EO und dem polyethoxylierten Methylglucosedistearat 20 EO ausgewählt ist.

8. Zusammensetzung nach einem der vorhergehenden Ansprüche,
dadurch gekennzeichnet, daß
die Fettsäure unter Palmitinsäure, Stearinsäure, Arachinsäure und Behensäure ausgewählt ist.

9. Zusammensetzung nach einem der vorhergehenden Ansprüche,
dadurch gekennzeichnet, daß
der lipophile grenzflächenaktive Stoff, der hydrophile grenzflächenaktive Stoff und die Fettsäure in einer Menge von 2 bis 6 Gew.-%, bezogen auf das Gesamtgewicht der Zusammensetzung, und vorzugsweise in einer Menge von 3 bis 4 Gew.-% enthalten sind.

10. Zusammensetzung nach einem der vorhergehenden Ansprüche,
dadurch gekennzeichnet, daß
der Anteil des lipophilen grenzflächenaktiven Stoffs, des hydrophilen grenzflächenaktiven Stoffs und der Fettsäure vorzugsweise im Bereich von 35 bis 55 Gew.-%, 25 bis 40 Gew.-% bzw. 15 bis 35 Gew.-%, bezogen auf ihr Gesamtgewicht, liegt.

11. Zusammensetzung nach einem der vorhergehenden Ansprüche,
dadurch gekennzeichnet, daß
der Anteil der umhüllten Ölkügelchen 5 bis 50 Gew.-%, bezogen auf das Gesamtgewicht der Zusammensetzung, vorzugsweise 10 bis 40 Gew.-%, beträgt.

12. Zusammensetzung nach einem der vorhergenden Ansprüche,
dadurch gekennzeichnet, daß
das Gewichtsverhältnis der Ölkügelchen zu den die Umhüllung bildenden grenzflächenaktiven Stoffen 2 bis 13, vorzugsweise etwa 7, beträgt.

13. Zusammensetzung nach einem der vorhergehenden Ansprüche,
dadurch gekennzeichnet, daß
das basische Mittel in der wäßrigen Phase in einer Menge aufgelöst ist, die mindestens so groß ist wie die Menge, die für die Neutralisation der Fettsäure erforderlich ist.

14. Zusammensetzung nach einem der vorhergehenden Ansprüche,
dadurch gekennzeichnet, daß
das basische Mittel unter Natriumhydroxid, Triethanolamin, Lysin und Arginin ausgewählt ist.

15. Zusammensetzung nach einem der vorhergehenden Ansprüche,
dadurch gekennzeichnet, daß
die wäßrige Phase zusätzlich einen oder mehrere kosmetische oder dermatologische hydrophile Wirkstoffe in freier oder verkapselter Form enthält.

16. Zusammensetzung nach Anspruch 15,
dadurch gekennzeichnet, daß
der kosmetische oder dermatologische hydrophile Wirkstoff in ionischen und/oder nichtionischen Lipidvesikeln oder Nanopartikeln, Nanokugeln, Nanoschwämmen oder Nanokapseln eingekapselt ist.

17. Zusammensetzung nach einem der vorhergehenden Ansprüche,
dadurch gekennzeichnet, daß
die Ölkügelchen mindestens eine Fettsubstanz oder lipophile Substanz mit Wirksamkeit bei der Hautpflege enthalten.

18. Zusammensetzung nach Anspruch 17,
dadurch gekennzeichnet, daß
die Fettsubstanz oder lipophile Substanz unter Antioxidantien, Mitteln gegen freie Radikale, Hydratisierungsmitteln, Melaninregulatoren, Bräunungsbeschleunigern, Depigmentierungemitteln, Hautfärbemitteln, fettregulierenden Mitteln, Schlankheitsmitteln, Mitteln gegen Akne, Antiseborrhöika, Mitteln gegen Hautalterung, Mitteln gegen Falten, UV-Schutzmitteln, Keratolytika, entzündungshemmenden Mitteln, kühlend wirkenden Mitteln, Wundheilungsmitteln, Gefäßschutzmitteln, antibakteriellen Mitteln, fungiziden Mitteln, Antischweißmitteln, Deodorants, Hautkonditioniermitteln, Immunmodulatoren, Nährstoffen und etherischen Ölen und Parfüms ausgewählt ist.

19. Zusammensetzung nach einem der Ansprüche 1 bis 16,
dadurch gekennzeichnet, daß
die Ölkügelchen eine Fettsubstanz oder lipophile Substanz mit Wirksamkeit bei der Haarpflege enthalten.

20. Zusammensetzung nach Anspruch 19,
dadurch gekennzeichnet, daß
die Fettsubstanz oder lipophile Substanz unter melaninregulierenden Mitteln, fettregulierenden Mitteln, Antiseborrhöika, Mitteln gegen das Altern, UV-Schutzmitteln, Keratolytika, antibakteriellen Mitteln, fungiziden Mitteln, Mitteln gegen Schuppen, Mitteln gegen Haarausfall, Haarfärbemitteln, Haarentfärbemitteln, Reduktionsmitteln für Dauerwelle, Haarkonditionierern und Nährstoffen ausgewählt ist.

21. Zusammensetzung nach Anspruch 18 oder 20,
dadurch gekennzeichnet, daß
die fette oder lipophile Substanz unter folgenden Verbindungen ausgewählt ist: D-α-Tocopherol, DL-α-Tocopherol, D-α-Tocopherylacetat, DL-α-Tocopherylacetat, Ascorbylpalmitat, Glyceriden von Vitamin F, Vitaminen der D-Gruppe, Vitamin D₂, Vitamin D₃, Retinol, Retinoleatern, β-Carotin, D-Panthenol, Farnesol, Farnesylacetat, Jojobaöl, an essentiellen Fettsäuren reichem Johannisbeeröl, 5-(n-Octanoyl)-salicylsäure, Salicylsäure, Alkylestern von α-Hydroxysäuren, wie z.B. Citronensäure, Milchsäure, Glykolsäure, asiatischer Säure, madekassischer Säure, Asiaticosid, dem Gesamtextrakt von Centella asiatica, β-Glycyrrhetinsäure, α-Bisabolol, 2-Oleoylamino-1,3 octadecan, Phytantriol, dem Sphingomyelin der Milch, Phospholipiden maritimer Herkunft, die reich an mehrfach ungesättigten essentiellen Fettsäuren sind, Ethoxyquin, Rosmarinextrakt, Melissenextrakt, Quercetin, dem Extrakt getrockneter Mikroalgen, etherischem Bergamotteöl, Octylmethoxycinnamat, Butylmethoxydibenzoylmethan, Octyltriazon, gelbem, braunem, schwarzem und rotem Eisenoxid sowie Titanoxiden mit einer Teilchengröße im Mikrometer- oder Nanometerbereich oder in umhüllter Form, 3-(3,5-Di-*tert*.-butyl-4-hydroxybenzylidert)-campher, 2-Benzotriazol-2-yl-4-methyl-6-[3-[1,3,3,3-tetramethyl-1-[(trimethylsilyl)oxy]disiloxanyl]-2-methylpropyl]phenol, perfluoriertem Öl und hyperoxidiertem Maisöl.

22. Zusammensetzung nach einem der vorhergehenden Ansprüche,
dadurch gekennzeichnet, daß
das Verhältnis Öl/Wasser höchstens 1 beträgt.

23. Verfahren zur Herstellung einer Zusammensetzung nach einem der vorhergehenden Ansprüche,
dadurch gekennzeichnet, daß
in einem ersten Schritt die Ölphase, die der lipophilen grenzflächenaktiven Stoff, den hydrophilen grenzflächenaktiven Stoff, die Fettsäure, den kosmetischen oder dermatologischen Wirkstoff enthält, und die wäßrige Phase, die das Neutralisationsmittel enthält, unter Rühren vermischt werden und in einem zweiten Schritt das erhaltene Gemisch einer auf dem Kavitationsprinzip beruhenden Homogenisierung unterzogen wird.

24. Verfahren nach Anspruch 23,
dadurch gekennzeichnet, daß
die auf dem Kavitationsprinzip beruhende Homogenisierung entweder unter hohen Drücken von 200 bis 1000 bar, mit Ultraschall oder mit Homogenisatoren, die mit einem Rotor/Stator-Kopf ausgerüstet sind, erreicht wird.

25. Verfahren nach einem der Ansprüche 23 und 24,
dadurch gekennzeichnet, daß
im ersten Schritt mindestens ein in freier Form vorliegender, kosmetischer oder dermatologischer Wirkstoff zugegeben wird.

26. Verfahren nach einem der Ansprüche 23 und 25,
dadurch gekennzeichnet, daß
in einem dritten Schritt mindestens ein verkapselt vorliegender, kosmetischer oder dermatologischer Wirkstoff zugemischt wird.

27. Öl-in-Wasser-Emulsion, die aus Ölkügelchen gebildet ist, die jeweils einzeln mit einer lamellaren, flüssigkristallinen Umhüllung versehen sind und in einer wäßrigen Phase dispergiert sind,
dadurch gekennzeichnet, daß
jedes Ölkügelchen einheitlich mit einer monollamellaren oder oligolamellaren Schicht umhüllt ist, die aus mindestens einem lipophilen grenzflächenaktivez, Stoff, mindestens einem hydrophilen grenzflächenaktiven Stoff und mindestens einer Fettsäure erhalten wurde, wobei die umhüllten Ölkügelchen einen mittleren Durchmesser unter 500 nm aufweisen, und daß die wäßrige Phase ein basisches Mittel in gelöster Form enthält.

28. Emulsion nach Anspruch 27,
dadurch gekennzeichnet, daß
das Verhältnis Öl/Wasser höchstens 1 beträgt.
